# EUROPEAN PATENT SPECIFICATION

(11) **EP 2 373 365 B1**
(45) Date of publication and mention of the grant of the patent: **23.04.2014**
(21) Application number: 09771818.3
(22) Date of filing: 07.12.2009
(51) Int. Cl.: A61M 5/32, A61M 5/42, A61M 5/46

(54) **ALIGNMENT OF A NEEDLE IN AN INTRADERMAL INJECTION DEVICE**
AUSRICHTUNG EINER NADEL IN EINEM INTRADERMALEN INJEKTIONSGERÄT
ALIGNEMENT D'UNE AIGUILLE DANS UN DISPOSITIF D'INJECTION INTRADERMIQUE

(30) Priority: 08.12.2008 US 201125 P; 22.12.2008 US 203274 P
(43) Date of publication of application: 12.10.2011
(73) Proprietor: Sid Technologies LLC, Newtown, PA 18940 (US); Program for Appropriate Technology in Health, Seattle, WA 98107 (US)
(72) Inventor: TSALS, Izrail, Newton, PA 18940 (US); HARVEY, Darrell, Seattle, WA 98115 (US)
(74) Representative: Lippert, Stachow & Partner
(86) International application number: PCT/US2009/066960
(87) International publication number: WO 2010/077596

(56) References cited:
- WO-A1-2008/131440
- US-A- 5 437 640
- US-A1- 2002 193 744

## Description

### BACKGROUND OF THE INVENTION

The invention relates to devices for delivery of intradermal injections generally, and more particularly to an adapter device combinable with a standard syringe to form an assembly for delivery of an intradermal injection.

Intradermal injections are used for delivering a variety of diagnostic and treatment compositions into a patient. Substances may be injected intradermally for diagnostic testing, such as to determine a patient's immunity status against tuberculosis and the status of allergic diseases. Vaccines, drugs and other compounds may also be delivered intradermally. In many instances, intradermal delivery is preferred because it generally requires a smaller volume dose of the diagnostic or treatment compound than other delivery techniques. An intradermal injection is made by delivering the substance into the epidermis and upper layer of the dermis. There is considerable variation in the skin thickness, both between individuals and within the same individual at different sites of the body. Generally the outer skin layer, or the epidermis, has a thickness between 500-200 microns and the dermis, the inner and thicker layer of the skin, has a thickness between 1.5-3.5 mm.

Making intradermal injections is difficult and generally requires an experienced nurse or medical professional. Incorrect placement of the tip of the needle cannula leads to a failed injection. The placement of the needle tip deeper than about 3.0 mm has the potential of delivering the injection into the subcutaneous region, where the intradermal dosage may be insufficient. Incorrect placement of the needle cannula may also puncture the skin again after being inserted into dermis, with the delivered compound being lost on the surface of the skin. Injection is often followed by a jet effect, with the compound exiting the injection site through the needle puncture track. The jet effect is even more pronounced for injections through a needle placed perpendicular to the injection site and in particular for shallow delivery. The success of intradermal injections is often determined by the experience of the healthcare professional. The preferred intradermal injection technique (using a standard needle) requires the healthcare professional to stretch the skin, orient the needle bevel to face upward, and insert a short bevel needle cannula at an angle of around 10-15 degrees, assuring that 2 to 3 mm of the needle cannula are located in the skin. The needle tip ends up positioned in the dermis or close to epidermis boundary. The compound is slowly injected into the skin of the patient, forming a blister or wheal. The insertion of the needle at an incorrect angle and/or depth results in a failed intradermal injection. Intradermal (ID) injection has been considered for immunization in the past, but has generally been rejected in favor of more reliable intramuscular or subcutaneous routes of administration because of the difficulty in making a successful ID injection.

Administration into the region of the intradermal space has been routinely used in the Mantoux tuberculin test, in which a purified protein derivative is injected at a shallow angle to the skin surface using a 27 or 30 gauge needle and a standard syringe. The technique is known to be quite difficult to perform and requires specialized training. A degree of imprecision in the placement of the injection results in a significant number of false negative test results. As a result, the Mantoux approach has not led to the use of intradermal injection for systemic administration of substances, despite the advantage of requiring smaller doses of substances.

There have been attempts to develop devices that would assure a precise needle penetration depth during ID injection which tends to vary due to tissue compliance, penetration angle, skill level and other factors. These are detailed in US Patent Numbers 4,393,870 and 6,200,291 and US Published Patent Applications Numbers 2003/0093032 and 2004/0147901. These devices employ complex constructions that tension the skin by vacuum, expanding the mounting surface prior to the needle insertion.

Alchas *et al.* developed a unique intradermal needle assembly for the delivery of compounds into the intradermal space by penetrating the dermis perpendicularly to its surface. A limiter supporting the needle is placed on the skin, the needle inserted, and the compound delivered. The penetration depth is in the 0.5 to 3 mm range, with a device limiter setting the penetration depth. There is a broad range of patents, issued and pending, defining different features of the system. U.S. Patent Numbers 6,494,865, 6,569,123, 6,689,118, 6,776,776, and U.S. Patent Publication Number 2003/0199822 describe such systems. The main limitation of the systems developed by Alchas et *al.* is the broad range of deposit depth due to assembly tolerances, needle bevel and the variations in skin properties. Another concern is back flow through the needle channel from the deposit pool to the surface of the skin due to a short direct channel formed by the needle. The jet effect further limits the performance when a shallow delivery is attempted.

The lack of suitable devices to accomplish reproducible delivery to the epidermal and dermal skin layers has limited the widespread use of the ID delivery route. Using conventional devices, ID injection is difficult to perform, unreliable and painful to the subject. There is thus a need for devices and methods that will enable efficient, accurate and reproducible delivery of agents to the intradermal layer of skin.

WIPO Patent Application Publication WO/2008/131440 ("the '440 publication") discloses devices and methods for intradermal administration of diagnostic and therapeutic agents, vaccines and other compounds into the dermal layer of the skin. The devices and the methods simplify the ID injection process and increase the consistency of the placement of the needle tip in the dermal space close to the skin surface allowing for a shallow cannula placement in the dermis. Furthermore, the devices and methods broaden the number of sites suitable for ID injection and make successful ID injections possible with limited training. However, the syringe is loaded from the rear and slides longitudinally into the adapter.

The applicability of devices disclosed in the '440 publication would be substantially broadened with design improvements allowing for improved placement depth of the cannula in the dermis. Furthermore, a design facilitating the ease of device and syringe merger would be of a substantial benefit. The improvements facilitating the ID injection using one hand and other features would also be beneficial for the use of ID devices and methods. There is thus a need for improvements to devices and methods for efficient accurate and reproducible delivery of agents to the intradermal layer of skin.

### BRIEF SUMMARY OF THE INVENTION

Briefly stated, in a first aspect the invention is an adapter device for use in combination with a syringe to form an assembly for delivering an intradermal injection. The adapter device comprises a body which is connectable to the syringe, the body being formed at least in part as a C-shaped tube to provide an opening for receiving at least a portion of a barrel of the syringe. At least one support element is connected to the body. With the assembly in an assembled condition, the at least one support element supports a needle cannula connected to the syringe. The needle cannula is supported by the at least one support element intermediate a base and a tip of the needle cannula. At least a terminal portion of the needle cannula extends axially in a direction at least substantially parallel to at least a planar portion of the second primary skin contacting surface.

Preferably, the adapter device further comprises a first primary skin contacting surface positioned at the distal end of the body. The first primary skin contacting surface is positioned at an angle to the second primary skin contacting surface between approximately 100 degrees and approximately 165 degrees.

The adapter device may preferably include a single support element connected to the body, wherein the support element supports the needle cannula at a point offset from a syringe centerline in one of a plane at least substantially perpendicular to the planar portion of the second primary skin contacting surface or a plane at least substantially parallel to the planar portion of the second primary skin contacting surface.

### BRIEF DESCRIPTION OF THE SEVERAL VIEWS OF THE DRAWINGS

The foregoing summary, as well as the following detailed description of the invention, will be better understood when read in conjunction with the appended drawings. For the purpose of illustrating the invention, there are shown in the drawings embodiments which are presently preferred. It should be understood, however, that the invention is not limited to the precise arrangements and instrumentalities shown.

In the drawings:
FIG. 1 is a side elevational view of an adapter device in accordance with a second preferred embodiment of the present invention, shown with a fixed needle syringe in axial alignment in preparation for assembly with the adapter device;
FIG. 2 is a rear perspective view of the adapter device and syringe of FIG. 1, shown in an assembled condition;
FIG. 3A is a schematic representation of a side view of a first needle alignment technique, capable of being incorporated into the adapter device of FIG. 1;
FIG. 3B is a schematic representation of an end view of the needle alignment technique of FIG. 3A;
FIG. 4A is a schematic representation of a top plan view of a second needle alignment technique, capable of being incorporated into the adapter device of FIG. 1; and
FIG. 4B is a schematic representation of an end view of the needle alignment technique of FIG. 4A.

### DETAILED DESCRIPTION OF THE INVENTION

Certain terminology is used in the following description for convenience only and is not limiting. The words "right", "left", "lower", "upper", "horizontal" and "vertical" designate directions in the drawings to which reference is made. The words "inwardly" and "outwardly" refer to directions toward and away from, respectively, the geometric center of the adapter device.

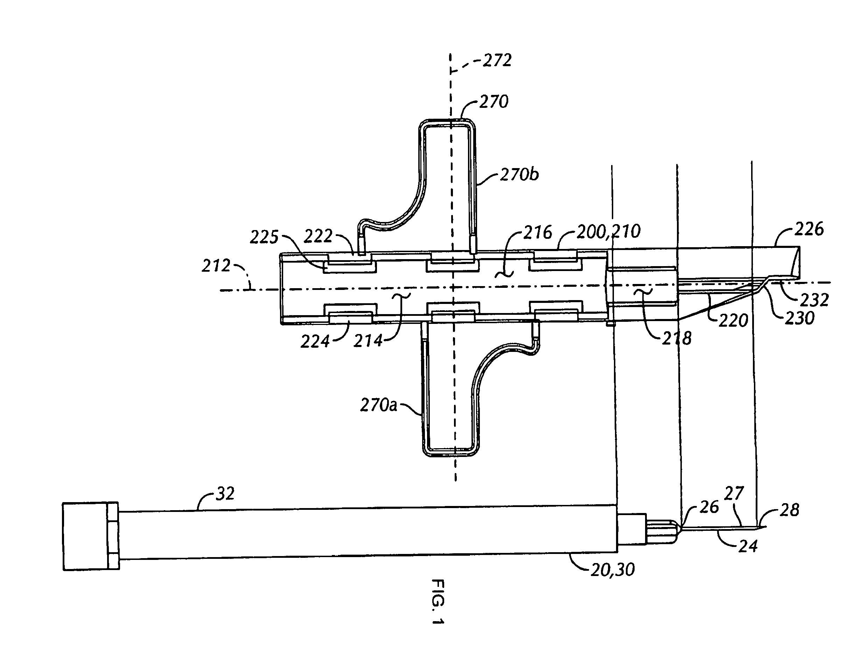

With reference now to FIG. 1, a presently preferred embodiment adapter device 200 is, intended for use in combination with a syringe 20 to form an assembly 10 for delivering an intradermal injection. The adapter device 200 comprises a body 210 connectable to the syringe 20. The body 210 is preferably formed in one piece as an integral, unitary component. The body 210 is preferably formed using conventional polymeric materials, such as polypropylene, using conventional fabrication techniques, for example, injection molding. Other known conventional materials and fabrication techniques could also be used.

The embodiment body 210 may be sized and shaped to accept any type of syringe 20, for example a fixed needle style syringe 30 shown in FIG. 1, or a Luer lock style syringe (not illustrated) or other standard syringes.

The syringe 20 has a centerline. A needle cannula 24 is typically disposed along the centerline, but could alternatively be positioned offset from the centerline. At a first, proximal end, the needle cannula 24 has a base 26 (see Fig. 1). at which the needle cannula joins a supporting hub. At a second, distal end, the needle cannula 24 a terminal portion 27 of the needle cannula, including a tip 28 (see Fig. 1), is inserted into a patient receiving the intradermal injection.

With reference now to FIG. 1, a presently preferred embodiment adapter device 200 is intended for use in combination with a syringe 20 to form an assembly 10 for delivering an intradermal injection. The adapter device 200 comprises a body 210 connectable to the syringe 20. The body 210 is preferably formed in one piece as an integral, unitary component. The body 210 is preferably formed using conventional polymeric materials, such as polypropylene, using conventional fabrication techniques, for example, injection molding. Other known conventional materials and fabrication techniques could also be used.

The body 210 may be sized and shaped to accept any type of syringe 20, for example a fixed needle style syringe 30 shown in FIG. 1, or a Luer lock style syringe (not illustrated) or other standard syringes.

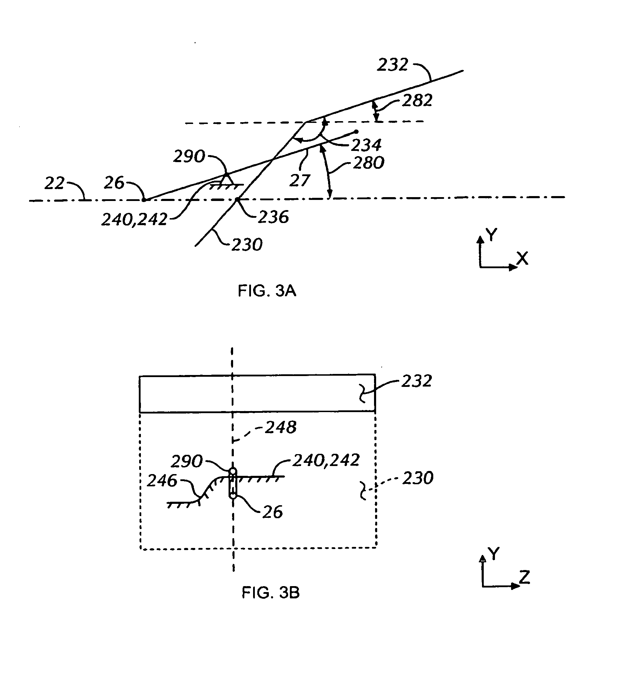

The body 210 has a body centerline 212, which, in the assembled condition, is preferably, but not necessarily, coincident with the syringe centerline. The body further comprises a second primary skin contacting surface 232 positioned at a distal end 226 of the body 210. The second primary skin contacting surface 232 is preferably, as illustrated, in its entirety a planar surface. Alternatively, the second primary skin contacting surface 232 could comprise both a planar surface forming only a portion of the second primary skin contacting surface 232, with a remaining portion of the second skin contacting surface being non-planar. In one embodiment, as illustrated in FIG. 1, the second primary skin contacting surface 232 is at least generally parallel to the syringe centerline and adapter body centerline 212. As discussed below relative to a second alignment technique illustrated in FIGS. 3A and 3B, at least the planar portion of the second primary skin contacting surface 232 could be inclined either up or down relative to the syringe and body centerlines 212.

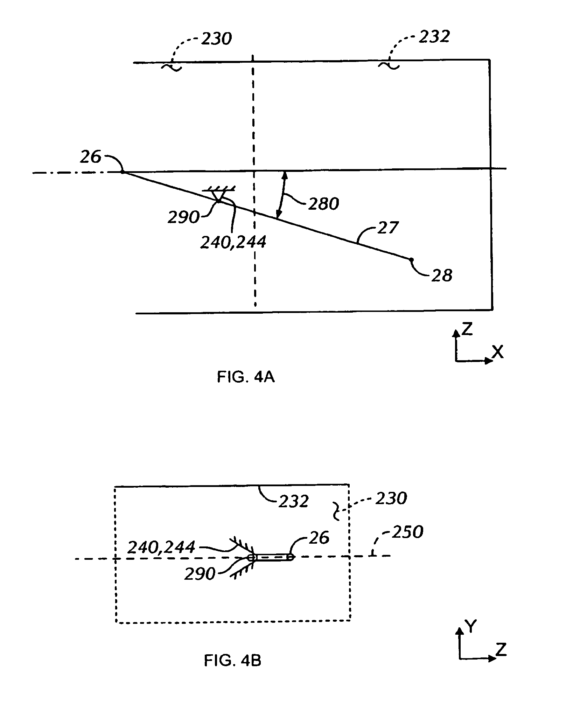

Preferably, the second embodiment adapter body 210 further comprises a first primary skin contacting surface 230 positioned at the distal end 226 of the body 210. Preferably the first and second primary skin contacting surfaces 230, 232 are positioned at an angle 234 (see FIG. 3A) between approximately 100 degrees and approximately 165 degrees. The first primary skin contacting surface 230 includes an opening 236 (illustrated only schematically in FIG. 3A) through which the needle cannula 24 extends through the first primary skin contacting surface 230. Note that the opening 236 must be sized sufficiently large to accommodate deflection of the needle illustrated schematically in FIGS. 3A and 4A.

Like the second primary skin contacting surface 232, the first primary skin contacting surface 230 is preferably planar in its entirety, as illustrated, but alternatively could comprise both a planar portion and a non-planar portion (not illustrated).

Note as before with the first embodiment adapter device 100, that the first primary skin contacting surface 230 is optional. Alternatively, the opening 236 could be enlarged to such an extent as to effectively eliminate the first primary skin contacting surface 230. This alternative configuration is not illustrated in the drawings.

The adapter body 210 is formed to receive the syringe 20 in a sideways motion. Stated otherwise, the body 210 includes side openings 214, including a syringe barrel side opening 216, a syringe hub portion side opening 218, and a needle cannula side opening 220. The syringe 20 can thus be assembled with the adapter device 200 in a direction substantially perpendicular to body centerline 212.

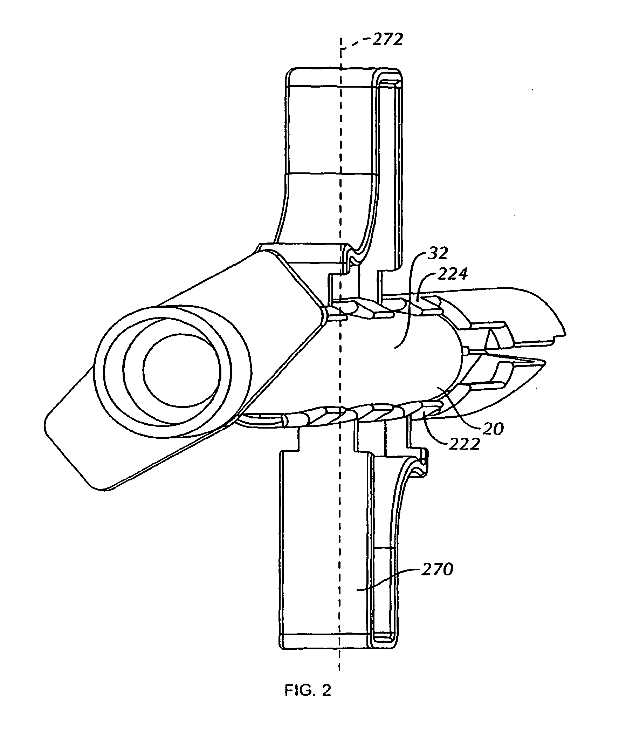

With reference now to both FIG. 1 and FIG. 2, the adapter body 210, is formed at least in part as a C-shaped tube 222 having gripping tabs 224. As best seen in FIG. 2, the gripping tabs 224 retain the syringe barrel 32 within the C-shaped tube 222. As the artisan skilled in the art of injection molding will appreciate, to facilitate the molding fabrication process, openings 225 are preferably provided to allow opposing molds to form the body 210, including the gripping tabs 224.

With continued reference to FIG. 1, the body 210 is further preferably provided with a pair of opposing finger flanges 270 to facilitate handling of the assembly during the injection process. First and second finger flanges 270a and 270b are configured to facilitate one-handed handling of the adapter device 200 during an injection. The finger flanges 270 are aligned along a central axis 272 which is at least substantially perpendicular to the planar portion of the second primary skin contacting surface 232.

Before proceeding further with the detailed description of the invention, it should be noted relative to the schematic representations of needle alignment techniques to be discussed herein below that the simple linear deflected shapes schematically illustrated do not accurately reflect the actual deflected shape of the needle cannula 24, which of course will vary depending on the support conditions and flexure characteristics of the actual needle cannula 24 (for example, stiffness of the needle hub, material of the needle cannula 24, and diameter of the needle cannula 24). Accordingly, the simplified linear shapes shown should be understood to be mere approximations.

With reference now to FIGS. 2A and 2B, the adapter body 210 preferably comprises a single support element 240 connected to the body 210. With the assembly in an assembled condition, the single support element 240 supports the needle cannula 24 connected to the syringe 20. The needle cannula 24 is supported at a point 290 positioned intermediate the needle cannula base 26 and the tip 28. The support element 240 functions to align the needle cannula 24 primarily relative to the second primary skin contacting surface 232 such that at least the terminal portion 27 of the needle cannula 24 extends axially in a direction substantially parallel to at least the planar portion of the second primary skin contacting surface 232. In general, the single support element 240 supports the needle cannula 24 at a support point 290 which is offset from the syringe centerline in either (a) a plane substantially perpendicular to the planar portion of the second primary skin contacting surface 232 or (b) a plane substantially parallel to the planar portion of the second primary skin contacting surface.

More particularly, as illustrated schematically in FIGS. 2A and 2B, a first needle alignment technique is based on providing the single support element 240 having a planar portion 242. With the syringe 20 and second embodiment adapter device 200 fully assembled, the planar portion 242 positions the needle cannula 24 at a known support angle 280 relative to the syringe centerline. More particularly, the single support element 240 supports the needle cannula 24 at the support point 290 which is offset from the syringe centerline in a vertical plane 248 substantially perpendicular to the planar portion of the second primary skin contacting surface 232. The offset can be either toward the second primary skin contacting surface 232, or away from it (that is, either in a positive or negative y direction, (the y direction being as indicated in FIGS. 3A and 3B)).

In order to appropriately control the depth of the injection, the planar portion of the second primary skin contacting surface 232 is inclined relative to the syringe centerline at a non-zero angle 282 (nominally equal to angle 280), such that the planar portion of the second primary skin contacting surface 232 is oriented substantially parallel to the terminal portion 27 of the needle cannula proximate the tip 28. The support angle 280 and the non-zero angle 282 are thus formed in the vertical plane 248 (illustrated in FIGS. 3A and 3B as the x-y plane) that is at least substantially perpendicular to the second primary skin contacting surface 232. Note that the non-zero angle 282 is inclined in a positive y direction if the support angle 280 is similarly inclined in a positive y direction (as illustrated in FIG. 3A), but if the support angle 280 were inclined in a negative y direction (not illustrated), the non-zero angle 282 would likewise be inclined in a negative y direction (not illustrated).

Recall that the simple linear deflected shape schematically illustrated does not accurately reflect the actual deflected shape of the needle. Accordingly, the actual optimal angle at which the second primary skin contacting surface should be inclined to the syringe centerline to accomplish as nearly as possible parallelism between the terminal portion 27 of the needle and the planar portion of the second primary skin contacting surface 232 may not be angle 280, but rather an angle approximately equal to support angle 280. In practice, the non-zero angle 282 may exceed the support angle 280 due to the fact that the needle cannula 24 will leave the hub along the syringe centerline and then be deflected at support point 290.

With reference to FIG. 3B, the needle cannula 24 is captured first by a ramp portion 246 which guides the needle cannula 24 to the planar portion 242 during the process of assembling the syringe 20 with the adapter device 200. A ramp design is needed in view of the uncertainty of where the needle cannula 24 is initially positioned. The ramp 246 extends sufficiently far from the syringe centerline such that the cone of uncertainty of where the needle cannula 24 is initially positioned is captured within the ramp portion 246. Note that the ramp portion 246 must terminate, and the planar portion 242 begin at a point at or beyond the cone of positional uncertainty such that when the needle cannula 24 is in its final supported position, the support point 290 falls along the planar portion 242.

Note that FIG. 3B illustrates assembly of the syringe 20 with the adapter 200 in a direction along the z axis (that is, "side insertion" along the horizontal axis). This same first alignment technique could be applied to an adapter (not illustrated) which receives the syringe in a direction along the y axis (that is, "bottom insertion" along the vertical axis). The needle cannula 24 will be deflected in a manner substantially similar to that illustrated in FIG. 3A, but in a negative y direction rather than a positive y direct. The planar portion of the second primary skin contacting surface 232 would likewise be inclined in a negative y direction. The planar support surface 242 would be positioned below the syringe centerline such that the support point 290 would fall at or outside of the cone of positional uncertainty. In this embodiment (not illustrated), two opposing ramps 246 could be provided to "funnel" the needle cannula 24 into position along a relatively narrow planar support surface 242, or alternatively the planar support surface 242 could be sufficiently broad to ensure contact with the needle cannula 24, irrespective of the needle cannula's initial position within the cone of positional uncertainty.

With reference now to FIGS. 4A and 4B, in a second needle cannula alignment technique, the support point 290 is offset from the syringe centerline in a horizontal plane 250 substantially parallel to the planar portion of the second primary skin contacting surface 232 (illustrated in FIG. 3A to be the x-z plane). In contrast to the first alignment technique, in the second alignment technique the planar portion of the second primary skin contacting surface 232 is oriented substantially parallel to the syringe centerline. In this second alignment technique, the support angle 280 is thus formed in the horizontal plane 250 that is at least substantially parallel to the second primary skin contacting surface 232. FIG. 4B illustrates that a single support element 240 having a V-groove portion 244 is used with this second needle cannula alignment technique. Similar to the concept behind sizing of the ramp portion 246 of the first alignment technique, the V-groove portion 244 is sized to capture the needle cannula 24 no matter where it is located within the cone of positional uncertainty associated with the expected range of angular straightness tolerance of the needle cannula 24.

As with the first alignment technique, it would be possible to reorient the V-groove portion 244 from a side insertion configuration (illustrated in FIGS. 4A and 4B) to a bottom insertion configuration (not illustrated), wherein the V-groove portion 244 is rotated 90 degrees, and the offset occurs in the vertical plane 248, which is at least substantially perpendicular to the horizontal plane 250. With such a reorientation, it would of course be necessary to also modify the orientation of the planar portion of the second primary skin contacting surface 232 relative to the syringe centerline such that the terminal portion of the needle cannula 24 is substantially parallel to the planar portion of the second primary skin contacting surface 232 in the assembled condition.

Rather than incorporating the V-groove portion 244 illustrated, alternatively two opposing inclined ramp portions (corresponding to opposing sides of the V-groove portion 244) could be provided, but rather than joining together at the vertex, the opposing sides could join opposing sides of a slot (not illustrated). The width of the slot could be slightly larger than the diameter of the needle cannula 24, to allow the needle cannula 24 to slide within the slot (not illustrated). The closed end of the slot would be positioned along the syringe centerline. Thus, the slot (not illustrated) would extend from approximately from where the vertex of the V-groove portion 244 would fall in the illustrated embodiment (see FIG. 4B) to the syringe centerline. Depending upon its initial position within the cone of positional uncertainty, the needle cannula 24 would be positioned at some point within the slot after assembly of the syringe 20 with the adapter device 200.

It will be appreciated by those skilled in the art that changes could be made to the embodiments described above without departing from the broad inventive concept thereof. It is understood, therefore, that this invention is not limited to the particular embodiments disclosed, but it is intended to cover modifications within the scope of the present invention as defined by the appended claims.

## Claims

1. An adapter device (200) connectable to a syringe (20) to form an assembly for delivering an intradermal injection, comprising:
a body (210) connectable to the syringe, the body (210) is formed at least in part as a C-shaped tube (222) to provide an opening (216) for receiving at least a portion of a barrel (32) of the syringe:
a second primary skin contacting surface (232) positioned at a distal end (226) of the body; and
at least one support element (240) connected to the body, wherein:
with the assembly in an assembled condition:
the at least one support element supports a needle cannula (24) connected to the syringe, with the needle cannula supported by the at least one support element intermediate a base (26) and a tip (28) of the needle cannula, and
at least a terminal portion (27) of the needle cannula proximate the tip extends axially in a direction at least substantially parallel to at least a planar portion of the second primary skin contacting surface (232).

2. The adapter device of claim 1, further comprising a first primary skin contacting surface (230) positioned at the distal end of the body and further positioned at an angle (234) to the second primary skin contacting surface (232) between approximately 100 degrees and approximately 154 degrees.

3. The adapter device of claim 1, wherein the syringe is a fixed needle style syringe (30).

4. The adapter device of claim 1, wherein the syringe is a Luer lock style syringe.

5. The adapter device of claim 1, further comprising a pair of finger flanges (270) connected to the body (210) wherein the finger flanges are aligned along a central axis (27) that is at least substantially perpendicular to a plane containing at least the planar portion of the second primary skin contacting surface (232).

6. The adapter device of claim 1, wherein:
a single support element (240) is connected to the body, and
the single support element supports the needle cannula (24) at a point (290) offset from a syringe centerline (22) in one of a plane (248) at least substantially perpendicular to the planar portion of the second primary skin contacting surface or a plane (250) at least substantially parallel to the planar apportion of the second primary skin contracting surface (232).

7. The adapter device of claim 6, wherein the point offset from the syringe (20) centerline is in the plane substantially perpendicular to the planar portion of the second primary skin contacting surface (232), and the planar portion of the second primary skin contacting surface is inclined relative to the syringe centerline at a non-zero angle (282) such that the planar portion of the second primary skin contacting surface is oriented substantially parallel to the terminal portion (27) of the needle cannula (24) proximate the tip (28).

8. The adapter device of claim 6, wherein the point offset from the syringe centerline is in the plane substantially parallel to the planar portion of the second primary skin contacting surface (232) and the planar portion of the second primary skin contacting surface is oriented substantially parallel to the syringe centerline.

9. The adapter device of claim 6, wherein the non-zero angle at least equals an expected range of angular straightness tolerance of the needle cannula (24).

10. The adapter device of claim 6, wherein a portion (242) of the single support element engaging the needle cannula (24) is planar and the non-zero angle is formed in the plane (248) that is at least substantially perpendicular to the second primary skin contacting surface (232).

11. The adapter device of claim 6, wherein a portion (244) of the support element engaging the needle cannula (24) has a V-groove shape and the non-zero angle is formed in the plane (25) that is a least substantially parallel to the second primary skin contacting surface (232).

## Patentansprüche

1. Adaptervorrichtung (200), die mit einer Spritze (20) verbindbar ist, um eine Einheit zur Verabreichung einer intradermalen Injektion zu bilden, umfassend:
einen mit der Spritze (210) verbindbaren Körper (210), der zumindest teilweise als C-förmiges Rohr (222) ausgebildet ist, um eine Öffnung (216) für die Aufnahme zumindest eines Abschnitts eines Kartuschenzylinders (32) der Spritze zu bilden;
eine zweite primäre Hautkontaktfläche (232), die an einem distalen Ende (226) des Körpers positioniert ist; und
mindestens ein Stützelement (240), das mit dem Körper verbunden ist; wobei:
mit der Einheit im zusammengesetzten Zustand
das mindestens eine Stützelement eine Nadelkanüle (24) stützt, die mit der Spritze verbunden ist, wobei die Nadelkanüle durch das mindestens eine Stützelement zwischen einer Basis (26) und einer Spitze (28) der Nadelkanüle gestützt wird und
wobei zumindest ein Endbereich (27) der Nadelkanüle in der Nähe der Spitze sich axial in einer Richtung erstreckt, die zumindest im Wesentlichen parallel zu zumindest einem ebenen Bereich der zweiten primären Hautkontaktfläche (232) ist.

2. Adaptervorrichtung nach Anspruch 1, ferner umfassend eine erste primäre Hautkontaktfläche (230), die an dem distalen Ende des Körpers und ferner unter einem Winkel (234) zu der zweiten primären Hautkontaktfläche (232) zwischen etwa 100 Grad und etwa 154 Grad positioniert ist.

3. Adaptervorrichtung nach Anspruch 1, wobei die Spritze eine Spritze vom Typ mit fester Nadel (30) ist.

4. Adaptervorrichtung nach Anspruch 1, wobei die Spritze eine Spritze vom Luer-Lock-Typ ist.

5. Adaptervorrichtung nach Anspruch 1, ferner umfassend ein Paar von Fingerflanschen (270), die mit dem Körper (210) verbunden sind, wobei die Fingerflansche entlang einer zentralen Achse (27) ausgerichtet sind, die zumindest im Wesentlichen senkrecht zu einer Ebene ist, die zumindest den ebenen Bereich der zweiten primären Hautkontaktfläche (232) enthält.

6. Adaptervorrichtung nach Anspruch 1, wobei:
ein einziges Stützelement (240) mit dem Körper verbunden ist und
wobei das einzige Stützelement die Nadelkanüle (24) an einem Punkt (290) stützt, der zur Spritzenmittellinie (22) in einer Ebene (248), die im Wesentlichen senkrecht zu dem ebenen Bereich der zweiten primären Hautkontaktfläche ist, oder einer Ebene (250), die zumindest im Wesentlichen parallel zu dem ebenen Bereich der zweiten primären Hautkontaktfläche (232) ist.

7. Adaptervorrichtung nach Anspruch 6, wobei der zur Mittellinie der Spritze (20) versetzte Punkt in der Ebene liegt, die im Wesentlichen senkrecht zu dem ebenen Bereich der zweiten primären Hautkontaktfläche (232) ist, und wobei der ebene Bereich der zweiten primären Hautkontaktfläche (232) relativ zur Mittellinie des Spritze unter einem Nichtnullwinkel (282) geneigt ist, derart, dass der ebene Bereich der zweiten primären Hautkontaktfläche im Wesentlichen parallel zu dem Endbereich (27) der Nadelkanüle (24) in der Nähe der Spitze (28) ist.

8. Adaptervorrichtung nach Anspruch 6, wobei der zur Mittellinie der Spritze versetzte Punkt in der Ebene liegt, die im Wesentlichen parallel zu dem ebenen Bereich der zweiten primären Hautkontaktfläche (232) ist, und wobei der ebene Bereich der zweiten primären Hautkontaktfläche im Wesentlichen parallel zur Mittellinie der Spritze orientiert ist

9. Adaptervorrichtung nach Anspruch 6, wobei der Nichtnullwinkel zumindest gleich einem angenommenen Winkel-Geradheitstoleranzbereich der Nadelkanüle (24) ist.

10. Adaptervorrichtung nach Anspruch 6, wobei ein Bereich (242) des einzigen Stützelements, der mit der Nadelkanüle (24) im Eingriff ist, eben ist und wobei der Nichtnullwinkel in der Ebene (248) gebildet ist, die im Wesentlichen senkrecht zur zweiten primären Hautkontaktfläche (232) ist.

11. Adaptervorrichtung nach Anspruch 6, wobei ein Bereich (244) des Stützelements, der mit der Nadelkanüle (24) im Eingriff ist, die Form einer V-Nut hat, und wobei der Nichtnullwinkel in der Ebene (25) gebildet ist, die im Wesentlichen parallel zur zweiten primären Hautkontaktfläche (232) ist.

## Revendications

1. Dispositif adapteur (200) attachable à une seringue (20), pour former un ensemble destiné à livrer une injection intradermique, le dispositif comprenant:
un corps (210) attachable à la seringue, corps qu'est formé au moins en partie comme un tube en forme de C, pour fournir une ouverture (216) destinée à recevoir au moins une partie d'un cylindre de cartouche (32) de la seringue;
une deuxième surface primaire de contact de peau (232) positionnée à une extrémité distale (226) du corps; et
au moins un élément de support (240) connecté au corps, dans lequel:
avec l'ensemble dans l'état assemblé:
ledit au moins un élément de support supporte une canule d'aiguille (24) connectée à la seringue, ladite canule d'aiguille étant supportée par ledit au moins un élément de support entre une base (26) et une pointe (28) de la canule d'aiguille, et
au moins une partie terminale (27) de la canule d'aiguille proche de la pointe s'étend axialement dans une direction au moins essentiellement parallèle à l'au moins une partie plate de la deuxième surface primaire de contact de peau (232).

2. Dispositif adapteur selon la revendication 1, comprenant en outre une première surface primaire de contact de peau (230) positionnée à une extrémité distale du corps et en outre positionnée dans un angle (234) par rapport à la deuxième surface primaire de contact de peau (232) entre 100 degrés et environ 154 degrés.

3. Dispositif adapteur selon la revendication 1, dans lequel ladite seringue est une seringue du type aiguille fixe (30).

4. Dispositif adapteur selon la revendication 1, dans lequel la seringue est du type Luer-Lock.

5. Dispositif adapteur selon la revendication 1, comprenant en outre un couple de flasques de doigt (270) connecté au corps (210), dans lequel les flasques de doigt sont alignées le long d'un axe central (27) qu'est au moins essentiellement perpendiculaire au plan qui contient au moins la partie plate de la deuxième surface primaire de contact de peau (232).

6. Dispositif adapteur selon la revendication 1, dans lequel:
un seul élément de support (240) est connecté au corps, et
ledit seul élément de support supporte la canule d'aiguille (24) sur un point décalé par rapport à une médiane de la seringue (22) dans un plan (248) au moins essentiellement perpendiculaire par rapport à la partie plate de la deuxième surface primaire de contact de peau ou par rapport à un plan (250) au moins essentiellement parallèle à la partie plate de la deuxième surface primaire de contact de peau (232).

7. Dispositif adapteur selon la revendication 6, dans lequel le point qui est décalé par rapport à la médiane de la seringue (20) est un plan essentiellement perpendiculaire par rapport à la partie plate de la deuxième surface primaire de contact de peau (232), et la partie plate de la deuxième surface primaire de contact de peau est inclinée par rapport à la médiane de la seringue dans un angle non nul (382) de sorte que la partie plate de la deuxième surface primaire de contact de peau est orientée essentiellement parallèlement par rapport à la partie terminale (27) de la canule d'aiguille (24) proche de la pointe (28).

8. Dispositif adapteur selon la revendication 6, dans lequel le point qui est décalé par rapport à la médiane de la seringue est dans le plan essentiellement parallèle à la partie plate de la deuxième surface primaire de contact de peau (232), et la partie plate de la deuxième surface primaire de contact de peau est orientée essentiellement parallèlement à la médiane de la seringue.

9. Dispositif adapteur selon la revendication 6, dans lequel l'angle non nul au moins est égal à une plage de tolérance de rectitude angulaire expectée de la canule d'aiguille (24).

10. Dispositif adapteur selon la revendication 6, dans lequel une partie (242) du seul élément de support qui engage la canule d'aiguille (24) est plate, et l'angle non nul est formé dans le plan (248) qui est essentiellement perpendiculaire par rapport à la deuxième surface primaire de contant de peau (232).

11. Dispositif adapteur selon la revendication 6, dans lequel une partie (244) de l'élément de support qui engage la canule d'aiguille (24) a une forme d'une rainure en forme de V, et l'angle non nul est formé dans le plan (25) qui est essentiellement parallèle à la deuxième surface primaire de contant de peau (232).
